# EUROPEAN PATENT APPLICATION

(11) **EP 1 327 678 A1**
(43) Date of publication of application: **16.07.2003**
(21) Application number: 01963415.3
(22) Date of filing: 05.09.2001
(51) Int. Cl.: C12M 1/42, C12N 5/00

(54) **METHOD AND APPARATUS FOR PEELING CELLS FROM VITAL TISSUE**

(30) Priority: 08.09.2000 JP 2000273005
(71) Applicant: Ajinomoto Co., Inc., Tokyo 104-8315 (JP)
(72) Inventor: UNEYAMA, Hisayuki, Ajinomoto Co., Inc., Kawasaki-shi, Kanagawa 210-8681 (JP); TORII, Kunio, Ajinomoto Co., Inc., Kawasaki-shi, Kanagawa 210-8681 (JP)
(74) Representative: Nicholls, Kathryn Margaret
(86) International application number: JP0107670
(87) International publication number: WO02020714

(57) **Abstract**

A method and an apparatus for peeling cells from a vital tissue by applying vibration to a part of the vital tissue have been developed.

For peeling cells from a vital tissue, a method for treating the whole vital tissue with an enzyme, a method for treating the whole vital tissue with chemicals or a method for applying vibration to the whole vital tissue has been so far employed. When cells peeled by the enzyme treatment method or the chemical treatment method are subjected to a pharmacological test or the like, a procedure of restoration from damages caused by the enzyme or the chemicals is needed after separation of the cells. Thus, it has been required to obtain cells as close to in-vivo cells as possible for a short period of time. Further, with respect to a method for peeling cells from a vital tissue by applying vibration to the whole vital tissue, the disclosure has been insufficient.

The invention provides a method and an apparatus for peeling cells close to in-vivo cells by applying vibration to a part of a vital tissue. Accordingly, the use of the cells peeled by the invention is quite effective for clarifying a biological structure, developing medications and the like.

## Description

### Technical Field

The present invention relates to a method for peeling cells from a vital tissue by applying vibration to a part of the vital tissue, and an apparatus for applying the vibration.

### Background Art

In recent years, studies on physiological functions and pharmacological studies using cells derived from vital tissues and cultured cells thereof have played an important role in the fields of medicine, agriculture, pharmacology, biology and the like. However, for peeling such cells from vital tissues, a method in which cells are peeled by treating the whole vital tissue with an enzyme, EDTA or the like to dissolve cell junctions, a method in which cells are physically peeled by applying vibration to the whole vital tissue, or the like has been so far a common practice.

It is considered that cells in a tissue making up a living body are firmly bound to one another by a binding agent called a cell binding factor. As a method for separating cells from a vital tissue, an enzyme treatment method in which a vital tissue is previously treated with an enzyme capable of dissolving each connective tissue, cells are peeled and dispersed with a pipette or a stirrer as required and a single cell and/or an aggregation of single cells is then separated is the most common practice. However, since an enzyme digests not only the connective tissue but also a part of a desired cell membrane, it is necessary that after the cells are isolated, these cells are incubated for a certain period of time for sufficient restoration from damages and then used in experiments (R. E. Numann et al., Neurosci. Lett. 47, 289 (1984)).

In a method for peeling a single esophageal epithelial cell, an esophageal epithelium is first collected, fragmented with a knife, and then digested with 0.025% trypsin at 37°C for 90 minutes while being shaken for dissolving a cell junction. Subsequently, the cell is physically peeled by pipetting (G. J. Brodmerkel, Gastroenterology, 60, 813 (1971)). In this pipetting, the vibration frequency is usually approximately 1 Hz, and this vibration is transmitted to the whole vital tissue of the fragment. Further, for peeling the cell by pipetting, it is required to sufficiently digest the cell connective tissue. Accordingly, even a part of a cell wall is digested by the trypsin treatment.

For example, in a method for peeling a single chicken embryonic fibroblast, an embryo collected from an egg is digested with 0.1% to 0.25% trypsin in a conical flask having a stirrer bar at room temperature and a moderate rotational speed for from approximately 10 to 20 minutes while being stirred. By this digestion, the cell is peeled (Shinsei Kagaku Jikken Koza (Tokyo Kagaku Dojin) 18 P. 130 (1990)). The rotational speed of this stirrer is from 100 to 200 rpm (Shinsei Kagaku Jikken Koza 18 P. 151 P. 154 (1990)), and the vibration by the rotation is transmitted to the whole vital tissue.

With respect to a nerve cell, the nerve cell is peeled by an enzyme treatment method using a collagenase or trypsin which is an enzyme suited for peeling a single nerve cell from a desired nerve nucleus (Shinsei Kagaku Jikken Koza 18 p. 170 (1990)). Nevertheless, a central nerve cell in particular is liable to damage. A nerve cell isolated from a small animal which is approximately 3 weeks old by an enzyme treatment method is destroyed within a few hours, and it is difficult to use the same in pharmacological studies or the like.

Meanwhile, when the foregoing enzyme treatment method is used as an in-vitro method for examining a function of drugs during nerve transmission, a connection between nerve cells (so-called a synaptic structure) is destroyed in a step of peeling a cell, making it impossible to observe a response of a presynapse. Accordingly, a brain slice specimen is used in screening. That is, there is no choice but to employ a procedure in which a brain of a small animal is extracted to prepare a thin brain slice specimen and an electric response of a nerve cell is measured by extracellular potential recording and intracellular potential recording. However, since the specimen is not a single cell, data analysis encounters difficulties by contamination in inserting a glass electrode or influence of other cells in measurement.

For measuring a response between synapses, it is considered that there is only a method using the foregoing brain slice specimen or a method in which a single nerve cell peeled is incubated for a few weeks to form a new synapse which is subjected to an experiment. In a culture system of a single nerve cell peeled by the latter enzyme treatment, the synapse newly formed is different from an in-vivo one owing to the enzyme treatment, and a synapse which is as close to an in-vivo synapse as possible is required.

Further, a method for peeling cultured cells and its apparatus (JP-A-58-158182) and a method for incubation and peeling and a peeling apparatus (JP-B-62-31910) are disclosed. Both of these relate to a method and an apparatus for peeling cells from an incubator by applying an impact force to the whole incubator. This method for peeling cells is for expediting the cell peeling depending on a difference in vibration frequency applied to the incubator and the cultured cells, and not a method for peeling cells from in-vivo cells. Moreover, the apparatus has a unit for applying the impact force to the whole incubator, but it does not have a function to impart the impact force to a part of a vital tissue. Accordingly, it is hard to effectively peel cells from a vital tissue using the very method and apparatus.

A method and an apparatus in which cells are peeled and extracted by applying a vibrational energy from outside to a surface of an internal organ are disclosed. Nevertheless, the vibrational energy is not transmitted to a part of a vital tissue, nor is the level of the vibrational energy disclosed (US 6054314). As a similar apparatus, an apparatus having a small metallic rod or plastic rod for transmitting rotation (10,000 rpm) of a micromotor rotating at a high speed is disclosed for peeling cells adhered to a small cavity of a cell incubator (FR 2537156). Since an object of this invention is to peel cells from the incubator, it is unclear whether or not the cells are peeled from an aggregation of cells. Only the rotational number is disclosed, and the vibrational energy thereof is unclear.

For peeling cells from hepatocytes in good yield, a method in which hepatocytes after perfusion are treated with an EDTA-containing Hank's solution and a peeling step is then performed in a peeling solution containing calcium chloride at a vibration frequency of 50 Hz for 60 seconds and a method in which after the similar treatment, vibration with a vibration frequency of from 50 to 90 Hz and an amplitude of 5 mm is applied for from 30 to 90 seconds is disclosed (SU 1463757, SU 1310426). However, since these procedures are methods in which cells are peeled from in-vivo cells that are easily peeled by a special treatment with EDTA, calcium chloride and the like to increase the yield of the resulting peeled cells, the cell walls undergo damages with chemicals. Moreover, the amplitude of the vibration applied is 5 mm, and the size of the in-vivo cell fragment is from 1 mm³ (cubic millimeter) to 3 mm³ (cubic millimeter). Therefore, the vibration is transmitted to the whole vital tissue, and an energy by which to peel cells is small.

A method and an apparatus for peeling cells by charging is disclosed (JP-A-10-42857). In the method and the apparatus, a cell binding material is negatively charged to conduct the peeling by negative charges of cells and the cell binding material, and this is not conducted with a vibrational energy. Further, according to this method, the whole vital tissue containing the cell binding material is charged.

As stated above, the disclosed techniques are the methods and/or apparatus in which the single cell and/or the aggregation of single cells is peeled from the vital tissue by the treatment with the enzyme or the chemicals such as EDTA. Further, they are the methods and/or apparatus in which the single cell and/or the aggregation of single cells is peeled by applying the physical energy such as vibration, charging or the like to the whole vital tissue. Still further, the level of the vibrational energy is not disclosed.

The foregoing prior techniques are to treat the whole vital tissue or to apply vibration, charging or the like to the whole vital tissue.

Akaike et al. disclose a novel method for peeling brain nerve cells except (1) a method in which enzyme treatment is used to peel brain nerve cells and (2) a method in which a brain slice specimen is used to examine an effect of a drug for a response between synapses, which are the ordinary common knowledges as mentioned above. In this method, a single nerve cell can be peeled from a desired nerve nucleus under an enzyme-free condition by applying vibration to a fragment of a vital tissue containing nerve cells. Besides, in the absence of an enzyme, it is possible to maintain a synaptic structure and separate a single nerve cell (synapse bouton specimen) having a presynapse adhered thereto. Thus, it is quite an excellent method in which a synaptic response can be measured with one single nerve cell without using a brain slice specimen. Its apparatus has a glass pipette with a smashed tip which allows vibration with a vibration frequency of from 3 Hz to 5 Hz and an amplitude of from 0.1 mm to 0.2 mm. It is disclosed that the glass pipette is gently placed on a brain fragment with a micromanipulator, and horizontally vibrated for 2 minutes to complete the cell peeling (N. Akaike, J. Physiology 518.2 525 (1999)).

The examination was conducted under the conditions disclosed by Akaike et al., but it was impossible to peel a single brain nerve cell.

### Disclosure of the Invention

For peeling cells from a vital tissue, a method for treating the whole vital tissue with an enzyme, a method for treating the whole vital tissue with chemicals or a method in which vibration is applied to the whole vital tissue has been so far employed. When cells peeled by the enzyme treatment method or the chemical treatment method are subjected to a pharmacological test or the like, a procedure of restoration from damages that the cells undergo with an enzyme or chemicals is needed after separation of the cells. Therefore, it has been required to obtain cells as close to in-vivo cells as possible for a short period of time. Moreover, with respect to a method for peeling cells from a vital tissue by applying vibration to the whole vital tissue, the disclosure has been insufficient.

The present inventors have found effective conditions for peeling cells from the vital tissue by applying vibration to a part of the vital tissue, and have developed an apparatus therefor. They have thus completed the invention.

A method for peeling cells from a vital tissue by applying vibration with a vibration frequency of from 10 Hz to 600 Hz and an amplitude of from 0.005 to 1 mm to a part of the vital tissue has been developed.

Examples of the vital tissue include vital tissues containing a brain nerve cell, a ganglion cell, an angioendothelial cell, a hepatocyte, an endocrine cell of a spleen or an adrenal, a gustatory cell, an ES cell, a fibroblast, an embryonic fibroblast, a fetal cell epidermal cell, an epithelial cell, a mammary gland cell, a muscle cell, a cartilage cell, a bone cell, a bone marrow cell, a cancer cell and a plant cell. However, these are not critical.

As the vital tissue, a vital tissue as such, a vital tissue subjected to chemical treatment, a vital tissue extracted from a living body as a tissue fragment, a tissue fragment subjected to enzyme treatment, culture treatment or chemical treatment, and a tissue fragment treated with a combination of these treatments are available. Further, a method for peeling cells in which a vibrational energy is applied to a part of the vital tissue can be performed under milder conditions with less influence on a cell membrane which have been so far employed, namely, conditions of low concentrations of an enzyme and chemicals, a short treatment time and/or a low treatment temperature.

The vibration is applied to a tissue fragment before the tissue fragment is subjected to enzyme treatment and chemical treatment, simultaneously with enzyme treatment, stationary culture treatment, agitation culture treatment and chemical treatment, or after these treatments.

When the vibration is applied to a certain point of the vital tissue as a center, it is not permitted to apply vibration except this vibration to a region of the vital tissue including the very center. In this region, the number of cells to which the vibration is applied is from 1 to 1,000, preferably from 5 to 200. The total number of cells in this region is at least twice, preferably at least five times, more preferably at least ten times as large as the number of cells to which the vibration is applied.

The vibration is applied to one single cell or two or more single cells in the vital tissue. The vibration frequency of this vibration is from 10 Hz to 600 Hz. It is preferably from 20 Hz to 300 Hz, more preferably from 30 Hz to 100 Hz. The amplitude of the vibration is from 0.005 mm to 1 mm. It is preferably from 0.05 mm to 0.5 mm, more preferably from 0.1 mm to 0.25 mm. The vibration frequency and the amplitude are not necessarily fixed during a peeling period of time. The vibration frequency and the amplitude may vary within the range of from 10 Hz to 600 Hz and the range of from 0.005 mm to 1 mm respectively. By applying the vibration to a part of the vital tissue, cells can be peeled also from the vital tissue which cannot be peeled by the method for peeling through pipetting or with a stirrer and the method for peeling in which the vibration or the charging is applied to the whole vital tissue.

The size of the single cell varies with the type of the cell, and its form is a polygonal, disk-like, cylindrical, fibrous, branched or spherical form. When a cell is schematically imagined as a smashed football in which the longest diameter of its form is defined as L, a diameter perpendicular to L as W, a height perpendicular to a surface of L x W as H and small W and H as a short diameter respectively, a single cell has the shortest diameter of approximately 0.002 mm and the longest diameter of approximately 0.1 mm. The amplitude applied to the vital tissue containing this single cell is from 0.5 time to 30 times, preferably from 1 time to 10 times the longest diameter, and from 1 time to 100 times, preferably from 10 times to 20 times the short diameter.

The cell can be peeled from the vital tissue by a cell peeling apparatus, characterized by having a unit for generating vibration with a vibration frequency of from 10 Hz to 600 Hz and an amplitude of from 0.005 mm to 1 mm and a unit for transmitting the vibration to a part of the vital tissue.

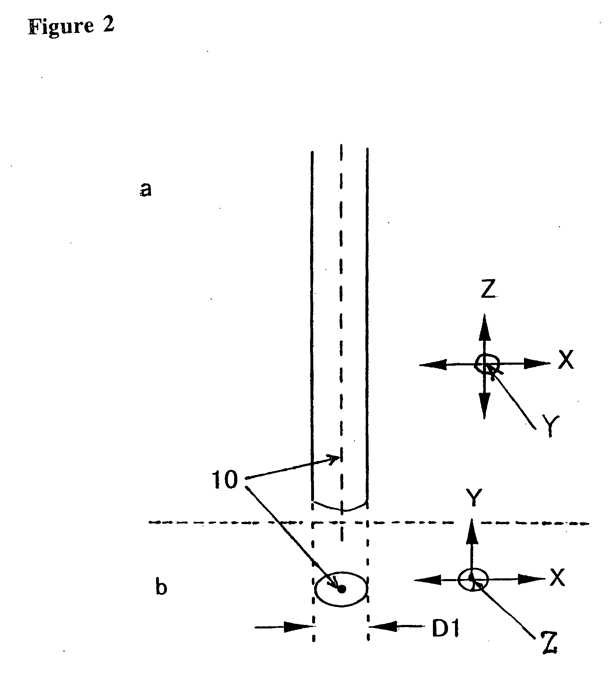

Examples of the method for applying the vibration to the vital tissue include a method in which the vibration is transmitted to the vital tissue by directly contacting a mechanically vibrating unit with the vital tissue, a method in which the mechanical vibration is transmitted via a solution or the like, a method in which the vibration with a low frequency is transmitted with a sound wave, a method in which a charge is applied to a cell to electrically transmit the vibration, a method in which the vibration is magnetically applied, and the like. In case of the vibration except the mechanical vibration, a directional sound wave, a directional on-off voltage and an electromagnetic wave can be used. There is a method using, as the vibration, single vibration, a combination of two or more vibrations different in a direction, vibration by rotation or a combination of any of these vibrations. Fig. 2 illustrates a portion which is contacted with the vital tissue. For example, the vibration may be vibration in an X direction, namely, vibration parallel to the vital tissue, vibration in a Z direction, namely, vibration perpendicular to the vital tissue, vibration by circular movement with an axis 10 as a rotational axis, or vibration by eccentric movement with an axis 10 as a center. Thus, the vibration is not particularly limited.

When the vibration is transmitted, a vibration transmitting site can be determined, as required, with a microscope or by monitoring the same with Scopeman (manufactured by Moritex Corporation) or a monitor similar to Scopeman.

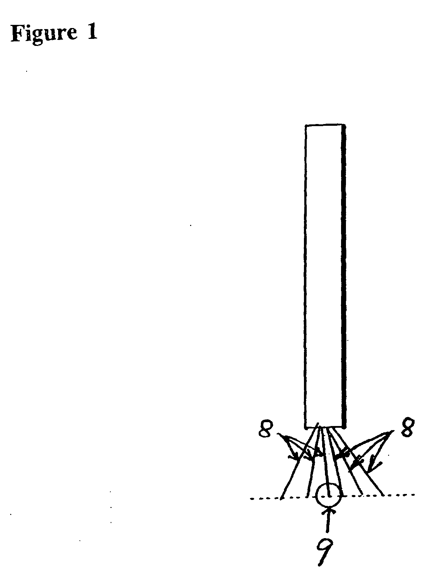

The number of the vibration transmitting site may be at least 1, and plural sites as shown in Fig. 1 are also available. In Fig. 1, 8 is a portion that is contacted with the vital tissue.

With respect to a portion in which the vibration is transmitted to the vital tissue, a glass, a metal, a metal oxide, ceramics, plastics or an optional combination thereof can be used as a material, and it may take the form of a rod, a rod with a stirring blade, a line, a plate, a powder, particles or an optional combination thereof. These are not critical. When the vibration is transmitted by contact with the vital tissue, an undamaged cell can be peeled in good yield by smoothening the contact portion unless the cell is mechanically destroyed by the contact portion.

Fig. 2 is an enlarged view of a portion 9 in Fig. 1. For example, in case of the contact portion shown in Fig. 2, its tip diameter D1 is from 0.001 mm to 4 mm, preferably from 0.01 mm to 1 mm, more preferably from 0.05 mm to 0.5 mm. Further, in case of using a magnetic force and an eddy current, a method is employed in which metallic grains suited therefor are used and added to a vessel charged with the vital tissue, and a magnetic force is generated from outside by on-off action, an eddy current is generated by on-off action or an on-off voltage is applied. The average grain size of the metallic grains is from 0.001 mm to 4 mm, preferably from 0.01 mm to 1 mm, more preferably from 0.05 mm to 0.1 mm.

When the vibration is transmitted to the vital tissue, the vibration frequency of the glass rod or the like is from 10 Hz to 600 Hz. It is preferably from 20 Hz to 300 Hz, more preferably from 30 Hz to 100 Hz. Further, the amplitude of the vibration is from 0.005 mm to 1 mm. It is preferably from 0.05 mm to 0.5 mm, more preferably from 0.1 to 0.25 mm. In some method for transmitting the vibration, the vibration frequency and the amplitude that the vital tissue experiences might be extremely decreased. In this case, the vibration generated by a device is adjusted while observing the peeling condition.

The time for applying the vibration varies with the objective vital tissue, the number of cells which are required to be peeled, the combined use with the enzyme treatment or the chemical treatment, the vibration frequency and the amplitude. It is usually from 10 seconds to 1 week, preferably from 30 seconds to 1 hour, more preferably from 60 seconds to 20 minutes.

When the vibration is applied, it can also be conducted while observing the vital tissue and the portion to which the vibration is applied with a microscope, a monitor camera, a digital camera, CCD or the like.

When a cell is isolated, a cell peeling device fitted with a pasteur's pipette is also useful.

When the vital tissue is referred to in the present specification, it indicates a part of a living body of animals or plants, a fragment extracted from the living body or a fragment extracted from the living body and subjected to pretreatment, enzyme treatment or chemical treatment.

When a part of the vital tissue is referred to in the specification, it indicates less than half of the vital tissue.

When the cell is referred to in the specification, it indicates a single cell and/or an aggregation of single cells.

When the single cell is referred to in the specification, it indicates one cell.

When the aggregation of single cells is referred to in the specification, it indicates an aggregation of 2 or more single cells.

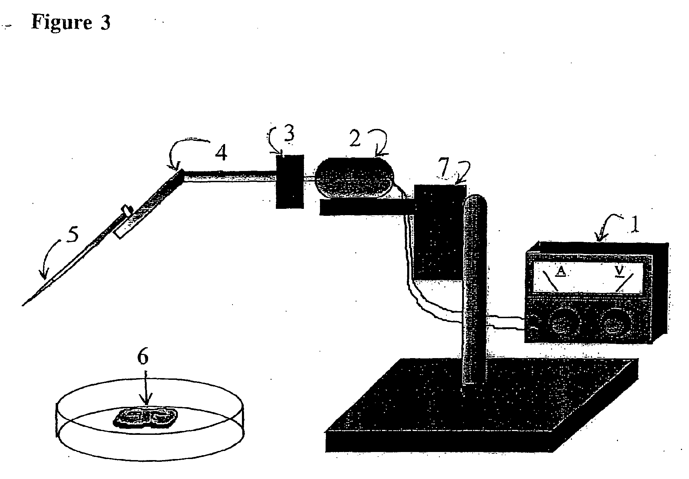

An example of the apparatus used in the invention is shown in Fig. 3. This apparatus has a vibration-generating portion including a power source unit 1, a servomotor 2 and a metallic vibration plate 3 in which the vibration frequency and the amplitude can freely be changed, a glass rod holder 4 portion for transmitting the vibration to a glass rod 5, and a glass rod 5 portion with a small tip for transmitting the vibration to a vital tissue 6. The glass rod 5 portion with the small tip is the same as shown in Fig. 2.

### Brief Description of the Drawings

Fig. 1 is a view showing a unit with plural contact portions for transmitting the vibration.
Fig. 2 is a view showing a unit for transmitting the vibration.
Fig. 3 is a schematic view of the apparatus.
Fig. 4 is a view showing a contact track of a portion that transmits the vibration.
Fig. 5 is a schematic view showing a differential interference microphotograph of a brain nerve cell.
Fig. 6 is a synapse transmission measuring chart of a brain nerve cell.

Reference numerals and symbols applied to the foregoing drawings indicate the following.
1 power source
2 servomotor
3 metallic plate
4 glass rod holder
5 glass rod
6 brain slice specimen
7 three-dimensional manipulator
8 portion in contact with a vital tissue
9 rod-like portion in contact with a vital tissue
10 central axis of a rod-like portion in contact with a vital tissue
   a side view of a rod-like portion in contact with a vital tissue
   b elevation of a rod-like portion in contact with a vital tissue
   c starting point of transmitting the vibration
   d end point of transmitting the vibration
   e synaptic terminal
   f brain nerve cell wall
   D1 diameter of a rod-like portion in contact with a vital tissue
   D2 diameter of a contact track pattern of a tip portion that transmits the vibration
   E enlarged distance photographed with a microscope
   F current value
   G time that lapses in measurement
   X, Y, Z coordinate axes

### Best Mode for Carrying Out the Invention

The invention is illustrated specifically below by referring to Examples and Reference Examples.

### (Example 1)

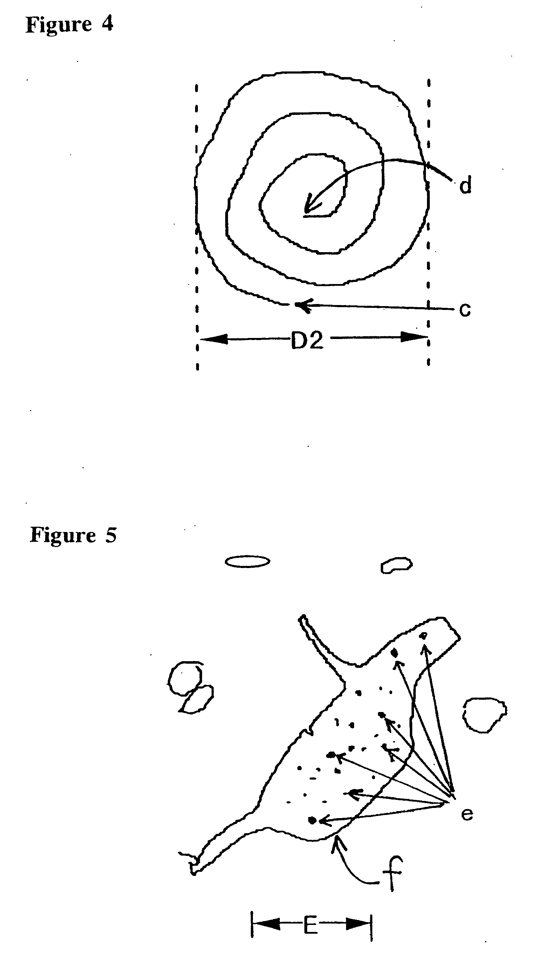

A Wister rat, 3 weeks old, was anesthetically dissected in a usual manner to form brain fragments. A thickness of the brain fragments is 0.5 mm. Of these, a fragment containing a hippocampus CA1 region was put on a petri dish. By using an apparatus shown in Fig. 3, a tip portion of a glass rod was contacted with a nerve nucleus portion containing a desired nerve cell by operation with a manipulator under a stereoscopic microscope with a 30x magnification, and the vibration was transmitted by moving the contact portion for 20 seconds from c as a starting portion to a point d through three rotations in a helical state with a diameter D2 of 1 mm as shown in Fig. 4. This operation was repeated three times in total for 60 seconds. At this time, in the tip of the glass rod, the vibration frequency was 10 Hz, the amplitude was 0.2 mm, and the total contact time was 1 minute. This vibration is in the X direction shown in Fig. 2.

Then, the magnification of the stereoscopic microscope was changed to 100x to measure the number of single cells peeled. This experiment is referred to as a first experiment. Further, a second experiment was performed as in the first experiment in a region of the brain fragment different from that in the first experiment to measure the number of single cells peeled. Still further, a third experiment was performed as in the experiment 1 in a region of the brain fragment different from those in the first and second experiments to measure the number of single cells peeled. As a result, the number of single cells peeled was 1 in the first experiment, 2 in the second experiment, and 5 in the third experiment.

### (Example 2)

The procedure in Example 1 was repeated. At this time, in the glass rod, the vibration frequency was 30 Hz, the amplitude was 0.2 mm, and the contact time was 1 minute. Subsequently, the number of single cells peeled was measured. As a result, it was 13 in the first experiment, 23 in the second experiment, and 16 in the third experiment.

### (Example 3)

The procedure in Example 1 was repeated. At this time, in the glass rod, the vibration frequency was 60 Hz, the amplitude was 0.2 mm, and the contact time was 1 minute. Subsequently, the number of single cells peeled was measured. As a result, it was 16 in the first experiment, 16 in the second experiment, and 25 in the third experiment.

### (Example 4)

The procedure in Example 1 was repeated. At this time, in the glass rod, the vibration frequency was 100 Hz, the amplitude was 0.2 mm, and the contact time was 1 minute. Subsequently, the number of single cells peeled was measured. As a result, it was 4 in the first experiment, 2 in the second experiment, and 3 in the third experiment.

### (Example 5)

After the completion of the experiment in Example 3, the brain fragment on the petri dish was removed to isolate the single nerve cells remaining on the bottom of the petri dish. By using the single nerve cells, it was confirmed by a patch clamp method that synaptic terminals were adhered. Further, the single nerve cell was photographed (400x magnification) with a differential interference microscope. A schematic view of the result was shown in Fig. 5. In Fig. 5, E is 0.02 mm. The result of photographing revealed that synaptic terminals e were adhered as shown in the schematic view also.

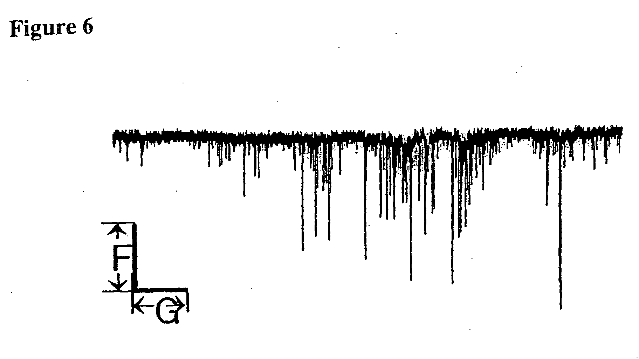

That the cell was the single nerve cell and the synapses were adhered was also clear from the results (Fig. 6) of measuring the current observed by a cell membrane voltage clamp method using a glass electrode. Incidentally, in Fig. 6, the current value F is 640.04 pA (picoampere), and a time that lapses in measurement is 6,553.6 mS (milliseconds).

### (Reference Example 1)

The procedure in Example 1 was repeated. At this time, in the glass rod, the vibration frequency was 1 Hz, the amplitude was 0.2 mm, and the contact time was 1 minute. Subsequently, the number of single cells peeled was measured. As a result, it was 0 in the first experiment, 0 in the second experiment, and 0 in the third experiment.

### (Reference Example 2)

The procedure in Example 1 was repeated. At this time, in the glass rod, the vibration frequency was 5 Hz, the amplitude was 0.2 mm, and the contact time was 1 minute. Subsequently, the number of single cells peeled was measured. As a result, it was 0 in the first experiment, 0 in the second experiment, and 0 in the third experiment.

### Industrial Applicability

The invention provides a method and an apparatus for peeling cells close to in-vivo cells by applying the vibration to a part of a vital tissue. Accordingly, the use of the cells peeled by the invention is quite useful in clarifying a biological structure, developing medications and the like.

## Claims

1. A method for peeling cells, **characterized by** applying vibration with a vibration frequency of from 10 Hz to 600 Hz and an amplitude of from 0.005 mm to 1 mm to a vital tissue to peel cells from the vital tissue.

2. The method for peeling cells, **characterized in that** the cells are peeled from the vital tissue according to the method of claim 1 in which the vibration frequency is from 20 Hz to 200 Hz.

3. The method for peeling cells, **characterized in that** the cells are peeled from the vital tissue according to the method of claim 1 in which the vibration frequency is from 30 Hz to 100 Hz.

4. The method for peeling cells, **characterized in that** the cells are peeled from the vital tissue according to the method of any one of claims 1 to 3 in which the amplitude is from 0.05 mm to 0.5 mm.

5. The method for peeling cells, **characterized in that** the cells are peeled from the vital tissue according to the method of any one of claims 1 to 3 in which the amplitude is from 0.1 mm to 0.25 mm.

6. A method for peeling nerve cells according to the method of any one of claims 1 to 5, **characterized in that** the vital tissue contains nerve cells.

7. A method for peeling brain nerve cells according to the method of any one of claims 1 to 5, **characterized in that** the vital tissue contains brain nerve cells.

8. An apparatus for peeling cells, **characterized by** comprising a unit for generating the vibration of any one of claims 1 to 5, and a unit for transmitting the vibration to a part of a vital tissue.
